(19) Europäisches Patentamt
European Patent Office
Office européen des brevets

(11) Publication number: 0 219 350 A2

# (12) EUROPEAN PATENT APPLICATION

(21) Application number: 86307976.0

(22) Date of filing: 15.10.86

(51) Int. Cl.[4]: C 07 C 93/08
C 07 C 93/10, A 61 K 31/135

(30) Priority: 16.10.85 GB 8525484

(43) Date of publication of application:
22.04.87 Bulletin 87/17

(84) Designated Contracting States:
AT BE CH DE ES FR GB GR IT LI LU NL SE

(71) Applicant: GLAXO GROUP LIMITED
Clarges House 6-12 Clarges Street
London W1Y 8DH (GB)

(72) Inventor: Finch, Harry
19 Hensley Close
Hitchin Hertfordshire (GB)

Lunts, Lawrence Henry Charles
10 Wormley West End
Broxbourne Hertfordshire (GB)

Naylor, Alan
35 Layston Park
Royston Hertfordshire (GB)

Skidmore, Ian Frederick
2 Wendover Drive
Welwyn Hertfordshire (GB)

(74) Representative: Marchant, James Ian et al
Elkington and Fife High Holborn House 52/54 High Holborn
London WC1V 6SH (GB)

## (54) Ethanolamine compounds.

(57) The invention provides compounds of the general formula (I)

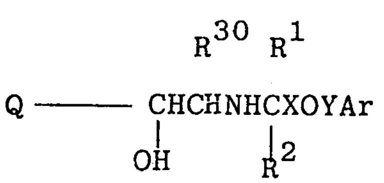

(I)

wherein
Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^4$ each represents a hydrogen atom, or a $C_{1-4}$ alkyl group, or $-NR^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R$ (where $R^7$ represents a $C_{1-4}$ alkyl, phenyl or $-NR^3R^4$ group), $-COR^8$ (where $R^8$ represents hydroxy, $C_{1-4}$alkoxy or $-NR^3R^4$, $-SR^9$ (where $R^9$ is a hydrogen atom, or a $C_{1-4}$ alkyl or phenyl group), $-SOR^9$, $-SO_2R^9$, or -CN, and q represents an integer from 0 to 3], or $-O(CH_2)_tR^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;
$R^1$ and $R^2$ each represent a hydrogen atom or a $C_{1-3}$ alkyl group, with the proviso that the sum total of carbon atoms in $R^1$ and $R^2$ is not more than 4;
$R^{30}$ represents a hydrogen atom or a $C_{1-3}$ alkyl group;
X represents a $C_{2-8}$ alkylene chain optionally substituted by one or two $C_{1-3}$ alkyl groups, or, when one carbon atom is substituted by two methyl groups, they may be linked to form an ethylene group, and
Y represents a $C_{1-7}$ alkylene chain optionally substituted by one or two $C_{1-3}$ alkyl groups, or, when one carbon atom is substituted by two alkyl groups, they may be linked to form an alkylene group; with the proviso that the sum total of carbon atoms in X and Y is 4 to 12; and with the further proviso that at least one of X and Y is substituted by one or two $C_{1-3}$ alkyl groups or an alkylene group; Q represents

EP 0 219 350 A2

(a) 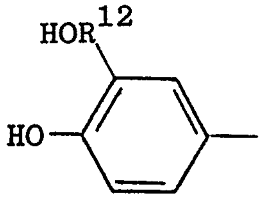

(where $R^{12}$ is a straight or branched $C_{1-3}$ alkylene group),

(b) 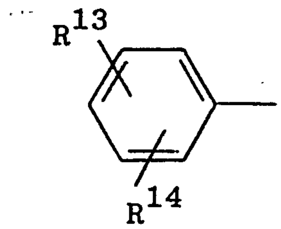

(where one or $R^{13}$ and $R^{14}$ is a hydroxy group and the other is a hydrogen or halogen atom or a hydroxy group),

(c) 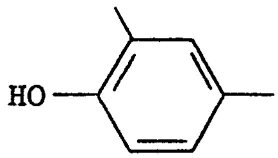

[where $R^{15}$ is a group $R^{16}CO$-, $R^{16}NHCO$-, $R^{16}R^{17}NSO_2$- or $R^{18}SO_2$- (where $R^{16}$ and $R^{17}$ is each a hydrogen atom or a $C_{1-3}$ alkyl group and $R^{18}$ is a $C_{1-3}$ alkyl group) and p is an integer 0 or 1],

(d) 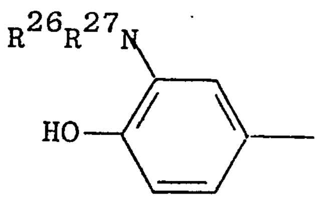

(where $R^{26}$ and $R^{27}$ is each a hydrogen atom or a $C_{1-3}$ alkyl group or, when $R^{26}$ is a hydrogen atom, $R^{27}$ may also be a $C_{1-4}$ alkoxycarbonyl group),

(e) 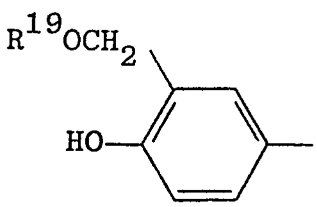

(where $R^{19}$ is a $C_{1-3}$ alkyl group),

(f) 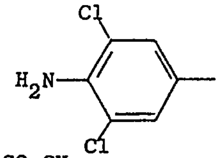

(g) 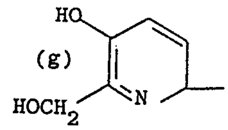

(h) 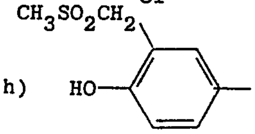

(i) 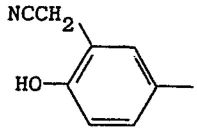

(j) 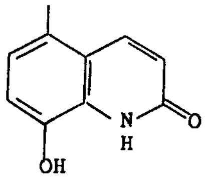

or

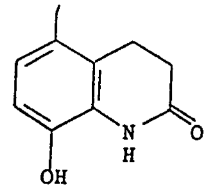

;

and physiologically acceptable salts and solvates (e.g. hydrates) thereof.

The compounds have a stimulant action at $\beta_2$-adrenoreceptors and may be used in the treatment of diseases associated with reversible airways obstruction such as asthma and chronic bronchitis.

**Description**

ETHANOLAMINE COMPOUNDS

This invention relates to ethanolamine compounds having a stimulant action at $\beta_2$-adrenoreceptors, to processes for their preparation, to pharmaceutical compositions containing them and to their use in medicine.

Ethanolamine derivatives of the general structure

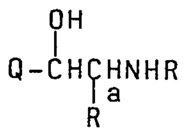

in which Q represents groupings of the type described hereinafter, $R_a$ represents inter alia a hydrogen atom or an alkyl group and, R represents inter alia an alkyl, aralkyl or aryloxyalkyl group have previously been described as bronchodilators having stimulant activity at β-adrenreceptors. We have now found a novel group of ethanolamine derivatives which differ in structure from those described previously, and have a desirable and potentially useful profile of activity.

Thus the present invention provides compounds of the general formula (I)

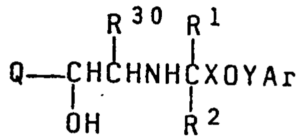

(I)

wherein

Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^4$ (where $R^3$ and $R^4$ each represents a hydrogen atom, or a $C_{1-4}$ alkyl group, or $-NR^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$alkoxy or $-NR^3R^4$), $-SR^9$ (where $R^9$ is a hydrogen atom, or a $C_{1-4}$ alkyl or phenyl group), $-SOR^9$, $-SO_2R^9$, or -CN, and q represents an integer from 0 to 3], or $-O(CH_2)_tR^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;

$R^1$ and $R^2$ each represent a hydrogen atom or a $C_{1-3}$ alkyl group, with the proviso that the sum total of carbon atoms in $R^1$ and $R^2$ is not more than 4;

$R^{30}$ represents a hydrogen atom or a $C_{1-3}$ alkyl group;

X represents a $C_{2-8}$ alkylene chain optionally substituted by one or two $C_{1-3}$ alkyl groups, or, when one carbon atom is substituted by two methyl groups, they may be linked to form an ethylene group, and

Y represents a $C_{1-7}$ alkylene chain optionally substituted by one or two $C_{1-3}$ alkyl groups, or, when one carbon atom is substituted by two alkyl groups, they may be linked to form an alkylene group; with the proviso that the sum total of carbon atoms in X and Y is 4 to 12; and with the further proviso that at least one of X and Y is substituted by one or two $C_{1-3}$ alkyl groups or an alkylene group;

Q represents

(a) 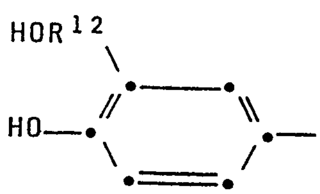

(where $R^{12}$ is a straight or branched $C_{1-3}$ alkylene group),

(b) 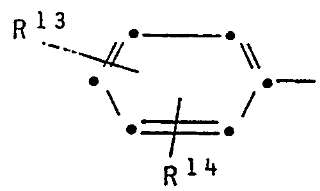

(where one of $R^{13}$ and $R^{14}$ is a hydroxy group and the other is a hydrogen or halogen atom or a hydroxy group),

(c) 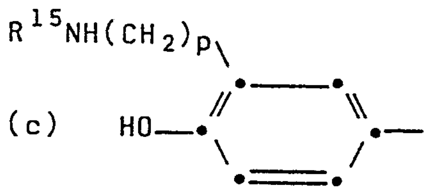

[where $R^{15}$ is a group $R^{16}CO-$, $R^{16}NHCO-$, $R^{16}R^{17}NSO_2-$ or $R^{18}SO_2-$ (where $R^{16}$ and $R^{17}$ is each a hydrogen atom or a $C_{1-3}$ alkyl group and $R^{18}$ is a $C_{1-3}$ alkyl group) and p is an integer 0 to 1],

(d) 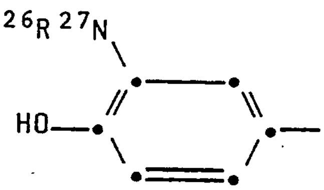

(where $R^{26}$ and $R^{27}$ is each a hydrogen atom or a $C_{1-3}$ alkyl group or, when $R^{26}$ is a hydrogen atom, $R^{27}$ may also be a $C_{1-4}$ alkoxycarbonyl group),

(e) 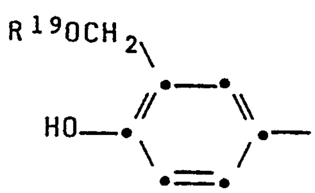

(where $R^{19}$ is a $C_{1-3}$ alkyl group),

(f) 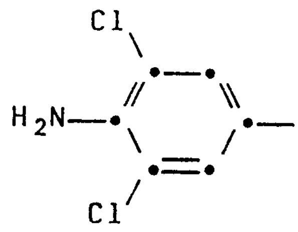 (g) 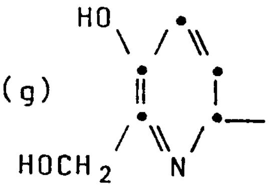

(h) 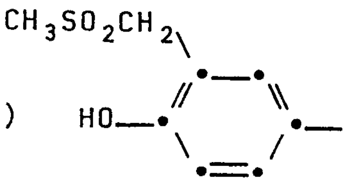 (i) 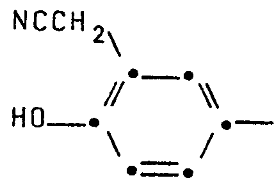

or

(j) 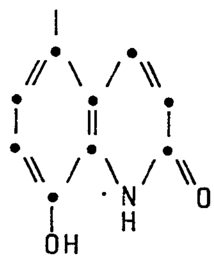 or 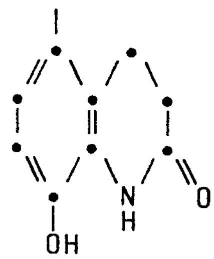 ;

and physiologically acceptable salts and solvates (e.g. hydrates) thereof.

It will be appreciated that the compounds of general formula (I) possess one or more asymmetric carbon atoms.

The compounds according to the invention thus include all enantiomers, diastereoisomers and mixtures thereof, including racemates. Compounds in which the carbon atom in the $-\underset{OH}{\underset{|}{CH}}-$ group is in the R configuration are preferred.

When $-NR^3R^4$ in compounds of formula (I) represents a saturated heterocyclic amino group this may be for example a pyrrolidino, piperidino, hexamethylenimino, piperazino, N-methylpiperazino, morpholino, homomorpholino or thiamorpholino group.

In the compounds of formula (I) Ar may be for example a phenyl group. Examples of the optional substituents which may be present on the phenyl group represented by Ar include chlorine, bromine, iodine, fluorine, methyl, ethyl, ethoxy, $-(CH_2)_qR$ [where R is hydroxy, methoxy, amino, methylamino, ethylamino, dimethylamino, diethylamino, morpholino, piperidino, piperazino, N-methylpiperazino, $-NHCOR^6$ (where $R^6$ is hydrogen or $C_{1-4}$alkyl (e.g. methyl, ethyl, isopropyl or n-butyl), $C_{1-4}$alkoxy (e.g. methoxy, ethoxy, isopropoxy or n-butoxy), phenyl, amino or N,N-dimethylamino), $-N(CH_3)COCH_3$, $-NR^5SO_2R^7$, (where $R^5$ represents a hydrogen atom or a methyl group and $R^7$ represents methyl, butyl, phenyl amino or dimethylamino), -COOH, $-COOCH_3$, $-COOCH_2CH_2CH_3$, $-CONH_2$, $-CON(CH_3)_2$, $-CON(CH_2CH_3)_2$, $-CON(CH_2CH_2CH_3)_2$, $-SR^9$ (where $R^9$ is methyl, ethyl or phenyl) $-SOCH_3$, $-SO_2CH_3$, or CN, and q is zero, 1, 2 or 3], $-NO_2$, $-O(CH_2)_2OH$, $-O(CH_2)_3OH$, $-O(CH_2)_2OCH_3$, or $-O(CH_2)_2OCH_2CH_3$.

The phenyl group represented by Ar may for example contain one, two or three substituents, which may be present at the 2-, 3-, 4-, 5- or 6-positions on the phenyl ring.

A preferred group of compounds are those in which Ar represents a phenyl group optionally carrying a single substituent.

In the compounds of formula (I) $R^1$ and $R^2$ may each be for example methyl, ethyl, propyl or isopropyl groups except that if one is an ethyl, propyl or isopropyl group the other is a hydrogen atom. Thus for example $R^1$ may be a hydrogen atoms or a methyl, ethyl or propyl group, and $R^2$ may be a hydrogen atom or a methyl group. $R^1$ and $R^2$ are each preferably a hydrogen atom or a methyl group.

A preferred group of compounds is that wherein $R^1$ and $R^2$ are both hydrogen atoms, or $R^1$ is a hydrogen atom and $R^2$ is a $C_{1-3}$ alkyl group, particularly a methyl group, or $R^1$ is a methyl group and $R^2$ is a methyl group.

Examples of the $C_{1-3}$ alkyl groups optionally present on the chains X and Y are methyl, ethyl, propyl or isopropyl groups.

The group X in formula (I) may for example contain 3 to 8 carbon atoms and the chain Y may contain for example from 2 to 7 carbon atoms.

Y may be for example $-(CH_2)_3-$,

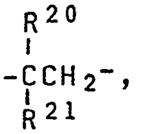

$-(CH_2)_2-$,

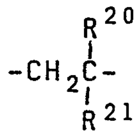, $-(CH_2)_4-$, 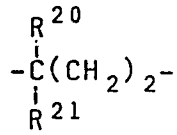, 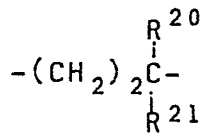, or 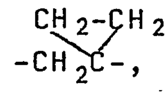

and X may be for example $-(CH_2)_3-$, $-(CH_2)_4-$,

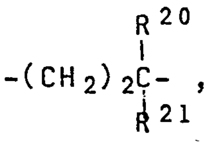

$-(CH_2)_5-$ ,

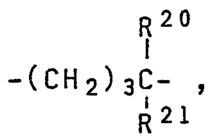 or 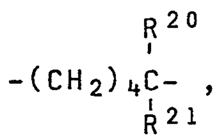

where $R^{20}$ is a methyl group and $R^{21}$ is a hydrogen atom or a methyl group.

Preferably the total number of carbon atoms in the chains X and Y is 6 to 12 inclusive and may be for example 7,8,9 or 10. Compounds wherein the sum total of carbon atoms in X and Y is 7,8 or 9 are particularly preferred.

Particularly preferred meanings for Y include $-(CH_2)_2-$,

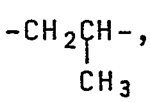 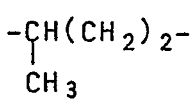 or 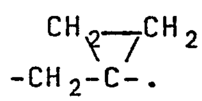

Particularly preferred meanings for X include $-(CH_2)_5-$and

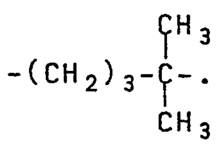

In the definition of Q in compounds for formula (I), $R^{12}$ may be, for example, $-CH_2-$, 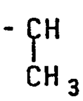, $-(CH_2)_2-$ or $-(CH_2)_3-$. "Halogen" in the definition of $R^{13}$ of $R^{14}$ may be for example chlorine or fluorine. $R^{16}$, $R^{17}$, $R^{26}$ and $R^{27}$ may each be, for example, a hydrogen atom or a methyl, ethyl, propyl or isopropyl group. $R^{18}$ may be a methyl, ethyl, propyl or isopropyl group. $R^{19}$ may be for example a methyl, ethyl or propyl group.

Q in compounds of formula (I) may be for example

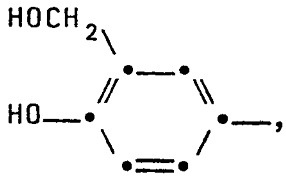,
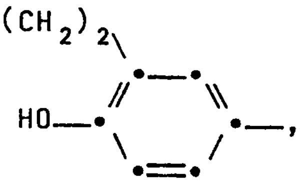,
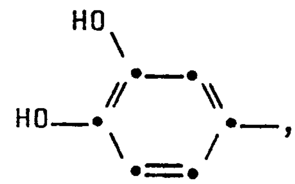,
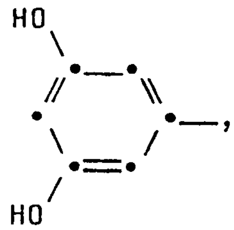,
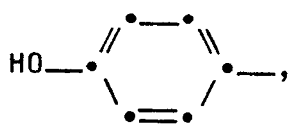,
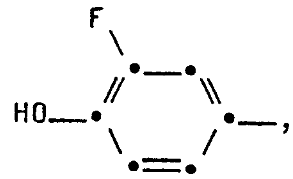,
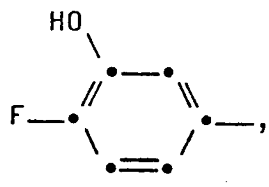,
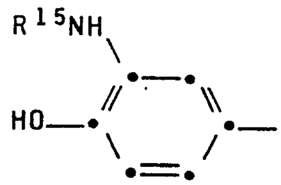 (where $R^{15}$ is HCO-, $CH_3CO$-, $NH_2CO$-, $(CH_3)_2NSO_2$- or $CH_3SO_2$-), 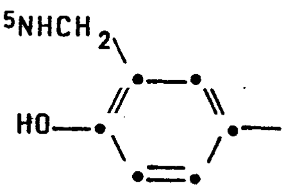 (where $R^{15}$ is as just defined), 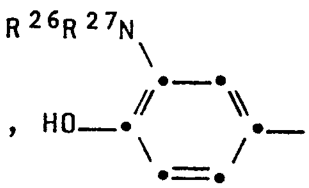 (where $R^{26}$ is hydrogen and $R^{27}$ is methyl),

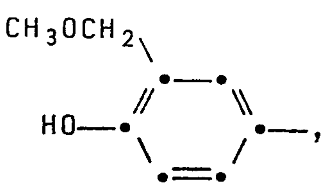, 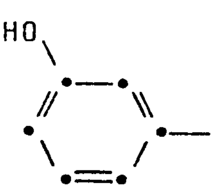

or a group of type (f), (g), (h), (i) or (j).

The group Q preferably represents the group

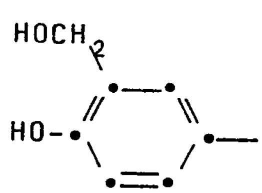

A particulalry preferred class of compounds according to the invention are those wherein Q represents the group

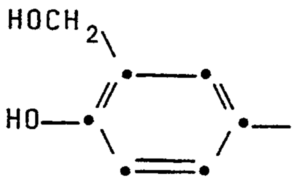

$R^1$, $R^2$ and $R^{30}$ each represent a hydrogen atom, Ar represents a phenyl group, X represents a group $-(CH_2)_5-$or

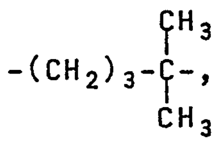

and Y represents a group 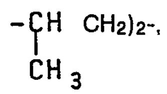

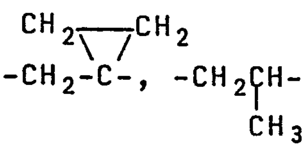 or $-(CH_2)_2-$.

From within this class of compounds a particularly preferred group of compounds are those wherein X represents $(CH_2)_5$ and X represents-$CH_2$ 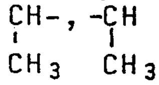-or

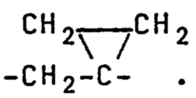 .

Suitable physiologically acceptable salts of the compounds of general formula (I) include acid addition salts derived from inorganic and organic acids, such as hydrochlorides, hydrobromides, sulphates, phosphates, maleates, tartrates, citrates, benzoates, 4-methoxy-benzoates, 2- or 4-hydroxybenzoates, 4-chlorobenzoates, p-toluenesulphonates, methanesulphonates, sulphamates, ascorbates, salicylates, acetates, fumarates, succinates, lactates, glutarates, gluconates, tricarballylates, hydroxy-naphthalenecarboxylates e.g. 1-hydroxy- or 3-hydroxy-2-naphthalenecarboxylates, or oleates. The compounds may also form salts with suitable bases. Examples of such salts are alkali metal (e.g. sodium and potassium), and alkaline earth metal (e.g. calcium or magnesium) salts.

The compounds according to the invention have a stimulant action at $\beta_2$-adrenoreceptors, which furthermore is of a particularly advantageous profile. The stimulant action was demonstrated in the isolated trachea of the guinea-pig, where compounds were shown to cause relaxation of PGF2$\alpha$-induced contractions. Compounds according to the invention have shown a particularly long duration of action in this test.

The compounds according to the invention may be used in the treatment of diseases associated with reversible airways obstruction such as asthma and chronic bronchitis.

The compounds according to the invention are also indicated as useful for the treatment of inflammatory and allergic skin diseases, congestive heart failure, depression, premature labour, glaucoma and in the treatment of conditions in which there is an advantage in lowering gastric acidity particularly in gastric and peptic ulceration.

The invention accordingly further provides compounds of formula (I) and their physiologically acceptable salts and solvates for use in the therapy or prophylaxis of diseases associated with reversible airways obstruction in human or animal subjects.

The compounds according to the invention may be formulated for administration in any convenient way. The invention therefore includes within its scope pharmaceutical compositions comprising at least one compound of formula (I) or a physiologically acceptable salt or solvate thereof formulated for use in human or veterinary medicine. Such compositions may be presented for use with physiologically acceptable carriers or excipients, optionally with supplementary medicinal agents.

The compounds may be formulated in a conventional manner in forms suitable for administration by inhalation or insufflation, or for oral, buccal, parenteral, topical (including nasal) or rectal administration.

Administration by inhalation or insufflation is preferred. The pharmaceutical compositions may be prepared by conventional means using physiologically acceptable examples.

A proposed daily dosage of active compound for the treatment of man is 0.005mg to 100mg, which may be conveniently administered in one or two doses. The precise dose employed will of course depend on the age and condition of the patient and on the route of administration. Thus a suitable dose for administration by inhalation is 0.005mg to 20mg, for oral administration is 0.02mg to 100mg, and for parenteral administration is 0.01mg to 2mg for administration by bolus injection and 0.01 mg to 25mg for administration by infusion.

The compounds according to the invention may be prepared by a number of processes, as described in the following. In describing the processes which may be used for preparing compounds of formula (I) and intermediates useful in the preparation thereof, Q, X, Y, $R^1$, $R^2$ and Ar are as defined for general formula (I) unless otherwise specified, or Ar may contain precursor substituent(s) convertible to the required substituent(s) by conventional means. It will be appreciated that certain of the reactions described below are capable of affecting other groups in the starting material which are desired in the end product; this applies especially in the reduction processes described, particularly where a hydride reducing agent is used and end-products are required containing the substituent $R^{16}CO$-, and where hydrogen and a metal catalyst are used in the preparation of compounds in which Q is the group

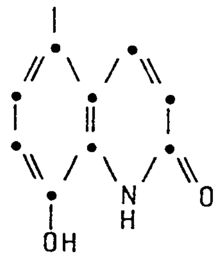

Care must therefore be taken in accordance with conventional practice, either to use reagents which will not affect such groups or to perform the reaction as part of a sequence which avoids their use when such groups are present in the starting material. In the preparation of both intermediates and end-products the final step in the reaction may be the removal of a protecting group. Suitable protecting groups and their removal are described in general process (2) below.

According to one general process (1), a compound of general formula (I) may be prepared by alkylation. Conventional alkylation procedures may be used.

Thus, for example, in one process (a), a compound of general formula (I) in which $R^1$ is a hydrogen atom may be prepared by alkylation of an amine of general formula (II)

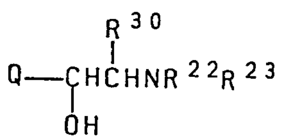 (II)

(wherein $R^{22}$ is a hydrogen atom or a protecting group, $R^{23}$ is a hydrogen atom, and any hydroxyl group(s) or amino group in the group Q may be protected) followed by removal of any protecting group where present.

The alkylation (a) may be effected using an alkylating agent of general formula (III):

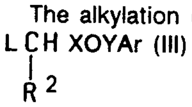 (III)

(wherein L represents a leaving group, for example a halogen atoms such as chlorine, bromine or iodine or a hydrocarbylsulphonyloxy group such as methanesulphonyloxy or p-toluenesulphonyloxy).

The alkylation is preferably effected in the presence of a suitable acid scavenger, for example, inorganic bases such as sodium or potassium carbonate, organic bases such as triethylamine, diisopropylethylamine or pyridine, or alkylene oxides such as ethylene oxide or propylene oxide. The reaction is conveniently effected in a solvent such as acetonitrile or an ether e.g. tetrahydrofuran or dioxan, a ketone e.g. butanone or methyl isobutyl ketone, a substituted amide e.g. dimethylformamide or a chlorinated hydrocarbon e.g. chloroform at a temperature between ambient and the reflux temperature of the solvent.

According to another example (b) of an alkylation process, a compound of general formula (I) in which $R^1$ represents a hydrogen atom may be prepared by alkylation of an amine of general formula (II) as previously defined except that $R^{23}$ is a hydrogen atom or a group convertible thereto under the reaction conditions, with a compound of general formula (IV):

$R^2COXOYAr$ (IV)

in the presence of a reducing agent, followed when necessary by removal of any protecting groups.

Examples of suitable $R^{23}$ groups convertible into a hydrogen atom are arylmethyl groups such as benzyl, α-methylbenzyl and benzhydryl.

Suitable reducing agents include hydrogen in the presence of a metal catalyst such as platinum, platinum

oxide, palladium, palladium oxide, Raney nickel or rhodium, on a support such as charcoal, using an alcohol, e.g. ethanol or an ester e.g. ethyl acetate or an ether e.g. tetrahydrofuran, or water, as reaction solvent, or a mixture of solvents, e.g. a mixture of two or more of those just described at normal or elevated temperature and pressure, for example from 20 to 100°C and from 1 to 10 atmospheres. Alternatively when one or both of $R^{22}$ and $R^{23}$ are hydrogen atoms, the reducing agent may be a hydride such as diborane or a metal hydride such as sodium borohydride, sodium cyanoborohydride or lithium aluminium hydride. Suitable solvents for the reaction with these reducing agents will depend on the particular hydride used, but will include alcohols such as methanol or ethanol, or ethers such as diethyl ether or tert-butyl methyl ether, or tetrahydrofuran.

When a compound of formula (II) where $R^{22}$ and $R^{23}$ are each hydrogen atoms is used, the intermediate imine of formula (V) may be formed:

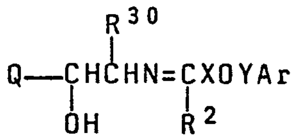

(V)

(where any hydroxyl or amino group(s) in Q may be protected).

Reduction of the imine using the conditions described above, followed, where necessary, by removal of any protecting groups, gives a compound of general formula (I).

Where it is desired to use a protected intermediate of general formula (II) it is particularly convenient to use hydrogen and a catalyst as described above with protecting group $R^{22}$ and any protecting group(s) in Q which are capable of being converted to a hydrogen atom under these reducing conditions, thus avoiding the need for a separate deprotection step. Suitable protecting groups of this type include arylmethyl groups such as benzyl, benzhydryl and α-methylbenzyl.

In another general process (2), a compound of general formula (I) may be obtained by deprotection of a protected intermediate of general formula (VI):

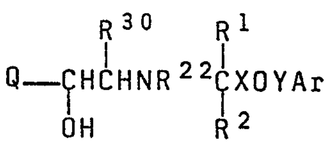

(VI)

(wherein $R^{22}$ and Q are as defined in formula (II) except that either $R^{22}$ is a protecting group and/or at least one of the hydroxyl or amino group(s) in Q is protected).

The protecting group may be any conventional protecting group, for example as described in "Protective Groups in Organic Chemistry", Ed. J.F.W. McOmie (Plenum Press, 1973). Examples of suitable hydroxyl protecting group(s) within the group Q are arylmethyl groups such as benzyl, diphenylmethyl or triphenylmethyl and tetrahydropyranyl. Examples of suitable amino protecting groups represented by $R^{22}$ or used for the amino group $R^{26}R^{27}N$ within Q are arylmethyl groups such as benzyl, α-methylbenzyl, diphenylmethyl or triphenylmethyl and acyl groups such as trichloroacetyl or trifluoroacetyl.

The deprotection to yield a compound of general formula (I) may be effected using conventional techniques. Thus for example, an arylmethyl group may be cleaved by hydrogenolysis in the presence of a metal catalyst (e.g. palladium on charcoal). When a hydroxyl group is protected as tetrahydropyranyl this may be cleaved by hydrolysis under acidic conditions. Acyl groups may be removed by hydrolysis, for example with a base such as sodium hydroxide, or a group such as trichloroacetyl may be removed by reduction with, for example, zinc and acetic acid.

In a particular embodiment of the deprotection process (2), a compound of general formula (VIII):

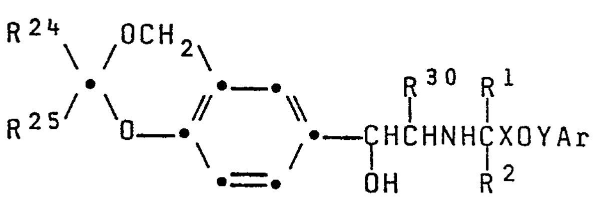

(VII)

(wherein $R^{24}$ and $R^{25}$, which may be the same or different, each represents a hydrogen atom or an alkyl or aryl group) may be deprotected.

A compound of general formula (I) may be obtained by treatment of a compound of formula (VII) with a dilute acid, for example hydrochloric acid in a solvent such as water or an alcohol such as ethanol at normal or

elevated temperature.

In another general process (3), a compound of general formula (I) may be prepared by reduction. Thus, for example, a compound of general formula (I) may be prepared by reducing an intermediate of general formula (VIII):

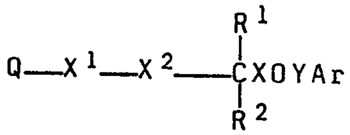

(VIII)

(wherein Q is as defined for general formula (II) and at least one of $X^1$ and $X^2$ represents a reducible group, and/or Q contains a reducible group and the other(s) take the appropriate meaning as follows, which is $X^1$ is -CH(OH)-, and $X^2$ is $-CHR^{30}NR^{22}-$ (where $R^{22}$ is hydrogen or a protecting group), followed where necessary by removal of any protecting groups.

Suitable reducible groups include those wherein $X^1$ is a group $>C=O$, $X^2$ is a group $-CHR^{30}NY^1-$ (wherein $Y^1$ represents a group convertible to hydrogen by catalytic hydrogenation, for example an arylmethyl group such as benzyl, benzhydryl or α-methylbenzyl), and Q contains a substituent -CHO, $-CO_2R^{28}$ (where $^{28}$ represents a hydrogen atom or an alkyl (e.g. $C_{1-3}$ alkyl) group or $-NR^{26}R^{29}$ where $R^{29}$ is an alkoxycarbonyl, aralkyloxycarbonyl, $C_{2-4}$alkanoyl or formyl group).

In one convenient aspect of the reduction process, any hydroxyl or amino group(s) with in Q may be protected as a group which is convertible to hydrogen under the reducing conditions employed and may be for example an arylmethyl group such as benzyl, benzhydryl or α-methylbenzyl.

The reduction may be effected using for example reducing agents conveniently employed for the reduction of carboxylic acids, aldehydes, esters, ketones and protected amines.

Thus for example when $X^1$ in general formula (VIII) represents a $>C=O$ group this may be reduced to a -CH(OH)-group using hydrogen in the presence of a catalyst as previously described for process (1) part (b). Alternatively, the reducing agent may be, for example, a hydride such as diborane or a metal hydride such as lithium aluminium hydride, sodium bis(2-methoxyethoxy) aluminium hydride (Red-Al), sodium borohydride or aluminium hydride. The reaction may be effected in a solvent, where appropriate an alcohol e.g. methanol or ethanol, or an ether such as tetrahydrofuran, or a halogenated hydrocarbon such as dichloromethane, at a temperature of 0°C to the refux temperature of the solvent.

When $X^2$ in general formula (VIII) represents a $-CHR^{30}NY^1-$ group this may be reduced to a $-CHR^{30}NH-$ group using hydrogen in the presence of a metal catalyst as previously described for process (1) part (b).

When Q in general formula (VIII) contains a substituents -CHO or $-CO_2R^{28}$ this may be reduced to a group $-CH_2OH$ using a hydride or complex metal hydride reducing agent as described above for the reduction of $>C=O$ to -CH(OH)-. When Q in general formula (I) contains a substituent $-NR^{26}R^{29}$ this may be reduced to a group $-NR^{26}R^{27}$ where $R^{27}$ represents an alkyl group using for example a complex metal hydride e.g. lithium aluminium hydride in a solvent such as an ether e.g. tetrahydrofuran.

It is also possible to prepare a compound of general formula (I) by a process comprising interconversion of one compound of general formula (I) into another compound of general formula (I).

Thus for example a compound of formula (I) in which Q represents the group

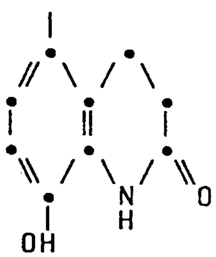

may be prepared by reduction of a corresponding compound of formula (I) in which Q is the group

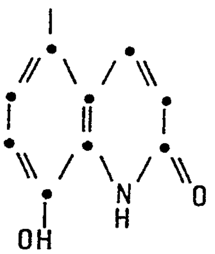

using for example hydrogen in the presence of a metal catalyst.

In the general processes described above, the compound of formula (I) obtained may be in the form of a salt, conveniently in the form of a physiologically acceptable salt. Where desired, such salts may be converted to the corresponding free acids using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or iso-propanol.

Physiologically acceptable salts may also be prepared from other salts, including other physiologically acceptable salts, of the compounds of general formula (I), using conventional methods.

When a specific enantiomer of a compound of general formula (I) is required, this may be obtained by resolution of a corresponding racemate of a compound of general formula (I) using conventional methods.

Thus, in one example an appropriate optically active acid may be used to form salts with the racemate of a compound of general formula (I). The resulting mixture of isomeric salts may be separated for example by fractional crystallisation, into the diastereoisomeric salts from which the required enantiomer of a compound of general formula (I) may be isolated by conversion into the required free base.

Alternatively, enantiomers of a compound of general formula (I) may be synthesised from the appropriate optically active intermediates using any of the general processes described herein.

Specific diastereoisomers of a compound of formula (I) may be obtained by conventional methods for example, by synthesis from an appropriate asymmetric starting material using any of the processes described herein, or by conversion of a mixture of isomers of a compound of general formula (I) into appropriate diastereoisomeric derivatives e.g. salts which then can be separated by conventional means e.g. by fractional crystallisation.

Intermediate compounds of general formula (VIII) for use in general process (3) may be prepared by a number of processes.

Thus for example intermediates of general formula (VIII) in which $X^1$ is a group $>C=O$ may be prepared from a haloketone of formula (IX)

$Q-COCHR^{30}Hal$ (IX)

(wherein any hydroxyl or amino group(s) in the group Q may optinally be protected) by reaction with an amine of general formula (X)

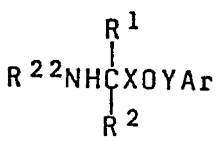 (X)

(wherein $R^{22}$ is a hydrogen atom or a group convertible thereto by catalytic hydrogenation).

The reaction may be effected in a cold or hot solvent, for example tetrahydrofuran, tert-butyl methyl ether, dioxan, chloroform, dimethylformamide, acetonitrile or a ketone such as butanone or methylisobutylketone, or an ester, for example ethyl acetate preferably in the presence of a base such a diisopropylethylamine, sodium carbonate or other acid scavenger such as propylene oxide.

Intermediates of general formula (VIII) in which $X^1$ is a group $>C=O$ may be reduced to the corresponding intermediate in which $X^1$ is a group -CH(OH)- using for example a metal hydride such as sodium borohydride in a solvent e.g. ethanol.

Amines of formula (II), haloketones of formula (IX), and intermediates of formulae (III), (IV) and )(X) are either known compounds or may be prepared by methods analogous to those described for the preparation of known compounds.

Suitable methods for preparing intermediates of formulae (III), (IV) and (X) are described in UK Patent Specificatin No. 2140800A and in the exemplification included hereinafter.

The following examples illustrate the invention. Temperatures are in °C. 'Dried' refers to drying using magnesium sulphate except where otherwise stated. Thin layer chromatograph (T.l.c.) was carried out over $SiO_2$. Flash column chromatography (FCC) was carried out on silica (Merck 9385) unless otherwise stated. The following abbreviations are used: DMF - dimethylformamide; THF -tetrahydrofuran; EA - ethyl acetate;

ER - diethyl ether.

Intermediate 1 referred to below is $\alpha^1$-(aminomethyl)-4-hydroxy-1,3-benzenedimethanol.

Intermediate 2

[2-[(6-Bromohexyl)oxy]-1-methylethyl]benzene

A mixture of β-methylbenzeneethanol (2g) 1,6-dibromohexane (11g), 50% aqueous sodium hydroxide (15ml) and tetrabutylammonium bisulphate (0.5g) was stirred at 23° for 24h. The mixture was diluted with water (100ml), extracted with ER (100ml) and the extract was washed with water (50ml), brine (50ml), dried and evaporated to give an oil. Purification by FCC eluting firstly with cyclohexane followed by cyclohexane - EA (4:1) afforded the title compound as a colourless oil (4.0g). T.l.c. (cyclohexane - EA 4:1) Rf 0.72.

Intermediate 3

N-[6-(1-Methyl-3-phenylpropoxy)hexyl]benzenemethanamine. hydrobromide

(i) 4-Phenylbutan-2-ol methanesulphonate

A solution of methanesulphonyl chloride (12.4mℓ) in tert. butyl methyl ether (ᵗBuOMe) (40mℓ) was added over 0.5h, at <5°, under $N_2$, to a stirred solution of 4-phenylbutan-2-ol (20g) and triethylamine (27.8mℓ) in ᵗBuOMe (160mℓ). The resulting suspension was stirred for 10min at 0°, 2M hydrochloric acid (14mℓ) and water (80mℓ) were added and the temperature allowed to rise to 20°. The organic phase was separated, dried and concentrated under reduced pressure to give the title compound as a colourless oil (30.79g).

Found: C,57.08;H,7.08S,13.70.

$C_{11}H_{16}SO_3$ requires C,57.87;H,7.06;S,14.04%.

(ii)6-(1-Methyl-3-phenylpropoxy)hexan-1-ol

Sodium hydroxide solution (35%w/v, 18.5mℓ) was added to a stirred mixture of 1,6-hexanediol (78.65g) and toluene (100mℓ) under nitrogen at 50°. Water was removed from the mixture by azeotropic distillation at atmospheric pressure whilst keeping the reaction under nitrogen. The product of stage (i) (30.5g) in ᵗBuOMe (60mℓ) was added to the toluene slurry over 0.5h at 80° under nitrogen. ᵗBuOMe and toluene were removed by vacuum distillation. The residue was cooled and diluted with water (100mℓ) at 60° and ᵗBuOMe (120mℓ) at 30°. The organic phase was separated and washed with brine (3x100mℓ) and water (2x100mℓ), dried, filtered and concentrated under reduced pressure to give the title compound as a colourless oil (20.75g).

Found: C,77.07;H,10.54.

$C_{16}H_{26}O_2$ requires C,76.75;H,10.47%.

(iii) 6-(1-Methyl-3-phenylpropoxy)hexane-1-ol methanesulphonate

A solution of methanesulphonyl chloride (7.8mℓ) in ᵗBuOMe (30mℓ) was added, over 0.5h, at 0-5°, under nitrogen, to a stirred solution of the product of stage (ii) (20.50g) and triethylamine (17.5mℓ) in ᵗBuOMe (120mℓ). The resulting suspension was stirred for 10min at 0°, warmed to 20° and acidified with 2M hydrochloric acid (10.5mℓ) in water (60mℓ). The organic phase was separated, dried, filtered and concentrated to an oil under reduced pressure to give the title compound as a colourless oil (27.36g).

Found: C,61.93;H,8.79;S,9.48.

$C_{17}H_{28}SO_4$ requires C,62.16;H,8.59;S,9.76%.

(iv) N-[6-(1-Methyl-3-phenylpropoxy)hexyl] benzenemethanamine hydrobromide

A mixture of the product of stage (iii) (27.3g), benzylamine (18.4mℓ), 35%w/v sodium hydroxide solution (31.6mℓ) and ᵗBuOMe (15mℓ) was stirred at 55° for 16h under nitrogen. The mixture was diluted with water (40mℓ) and methylisobutyl ketone (MIBK) (120mℓ) at 55°. The organic layer was separated at 50° and acidified to pH 1 with 47%v/v hydrobromic acid (21mℓ) and water (50mℓ) at 55°. The organic layer was separated and washed with water (3x75mℓ) at 55°, dried, filtered and concentrated under reduced pressure to give an oily solid. The solid was crystallised from hot MIBK/60-80° petroleum ether (30mℓ/60mℓ), the slurry was cooled to 5° and the solid filtered off. The product was triturated with refluxing 60-80° petrol ether (60mℓ) (+2%v/v MIBK), the slurry cooled to 20° and again filtered to give the title compound as a waxy solid (18.21g) m.p. 126-131°.

Intermediate 4

[1-[[(6-Bromohexyl)oxy]methyl]cyclopropyl]benzene

1-Phenylcyclopropanemethanol (2.50g) and 1,6 dibromohexane (12.37g) were stirred rapidly at room temperature with tetra-n- butylammonium bisulphate (0.49g) and 12.5M aqueous sodium hydroxide (16ml), for 18h. The mixture was diluted with water (65ml), extracted with ether (3x75ml) and the combined organic extracts were washed consecutively with water (65ml), brine (65ml), dried and evaporated to give an oil (11.51g). The oil was purified by FCC eluting with ER-cyclohexane (0:100→5:95) to give an oil (4.43g). This was further purified by FCC eluting with ER-cyclohexane (1:99→ 2:98) to give the title compound (3.83g) as a colourless oil. T.l.c. $SiO_2$ (1:99 ER-cyclohexane) Rf 0.20.

Intermediate 5

[2-[[5-Iodo-1,1-dimethylpentyl]oxy]ethyl]benzene

A mixture of 6-iodo-2-methyl-2-hexene (2.0g), phenethyl alcohol (2.14g) and concentrated sulphuric acid (0.5g) was allowed to stand room temperature for 45h. The dark coloured viscous oil was taken up in ether (75mℓ), the solution washed with 8% sodium bicarbonate solution (2x50mℓ) and dried ($MgSO_4$). Concentration in vacuo at 40° afforded the crude product which was purified on a column of 'flash' silica (Merck 9385; 15x5cm) elution with ether/hexane (1 :40) providing the title compound as a colourless oil (0.75g).
T.l.c. $SiO_2$ ($Et_2O$/hexane 1:25) Rf 0.43.

Example 1

4-Hydroxy-α[1] [[[6-(2-phenylpropoxy)hexyl]amino]methyl]-1, 3-benzenedimethanol

Intermediate 2 (1.0g) was added over 10min to a stirred solution of Intermediate 1 (0.92g) and N,N-diisopropylethylamine (0.7g) in DMF (15ml) at 80°. The mixture was diluted with water (100ml), extracted with EA (2x50ml), and the extract was washed with brine (50ml), dried ($Na_2SO_4$) and evaporated to give an oil. Purification by FCC on triethylamine deactivated silica (80g) eluting with EA-methanol (4:1) gave an oil which on trituration with cold ER gave the title compound as a beige semi-solid (0.35g). T.l.c. (EA-methanol 4:1) Rf 0.33.
Found: C,71.9;H,8.9;N,3.4.
$C_{24}H_{35}NO_4$ requires C,71.8;H,8.8;N,3.5%.

Example 2

4-Hydroxy-α[1]-[[[6-(1-methyl-3-phenylpropoxy)hexyl]amino] methyl]-1,3-benzenedimethanol

A solution of 2-bromo-1-[4-hydroxy-3-(hydroxymethyl) phenyl]ethanone (1.5g), N-[6-(1-methyl-3-phenylpropoxy) hexyl]benzenemethanamine (2.0g, generated from Intermediate 3), N,N-diisopropylethylamine (1.55g) and THF (30mℓ) was left at room temperature for 18h, filtered, and evaporated. The residue was purified for FCC eluting with ER to give a yellow oil (2.7g). The oil in ethanol (150mℓ) was hydrogenated over 10% palladium on charcoal (0.8g) and 5% platinum on charcoal (0.6g) for 5h, filtered, and evaporated. The residue was purified by FCC eluting with toluene - ethanol - ammonia (80:20:1) to give a colourless gum, which was triturated with ER (20mℓ) to give the title compound as an off-white solid (1.2g) m.p. 48-54°.
T.l.c. $SiO_2$ (toluene-ethanol-ammonia 80:20:1) Rf 0.2.

Example 3

4-Hydroxy-α[1]-[[[6-[(1-phenylcyclopropyl)methoxy]hexyl] amino]methyl]-1,3-benzenedimethanol.

A mixture of Intermediate 1 (1.03g), Intermediate 4 (1.00g), and N,N- diisopropylethylamine (1.17ml) in dry DMF (9.0ml) was heated at 80° for 1h under nitrogen. The clear brown solution was diluted with water (90ml), acidified to $pH_4$ with 2N hydrochloric acid and then basified to pH8 with solid potassium bicarbonate. The cloudy aqueous phase was extracted with EA (2x90ml) and the combined organic extracts were washed with water (90ml) and brine (45ml). The combined, dried ($Na_2SO_4$) extracts were evaporated and the recovered oil (1.12g) was purified by FCC eluting with EA-methanol-triethylamine (90:10:1). The recovered oil was triturated with cyclohexane (25ml) to give a solid (260mg) which was further purified by chromatography as above, and trituration, to give the title compound as a yellow oil (145mg). T.l.c. (EA-methanol-triethylamine 90:10:1) Rf 0.17.
Found: C,71.1;H,8.55;N,3.35.
$C_{25}H_{35}NO_4.O.37H_2O$ requires C,71.45;H,8.55;N,3.35%.

Example 4

4-Hydroxy-α[1]-[[[5,5-dimethyl-5-(2-phenylethoxy)pentyl]-amino]methyl]-1,3-benzenedimethanol

Intermediate 5 (0.61g) in DMF (3mℓ) was added dropwise over 10 min to a stirred solution of Intermediate 1 (0.64g) and N,N-diisopropylethylamine (0.68g, 0.91mℓ), in DMF (12mℓ) at 80° under nitrogen. The mixture was stirred at 80° for a further 2h, cooled, and the solvent removed in vacuo at 55°. The residual oil was partitoned between ethyl acetate (75mℓ) and water (50mℓ) and the aqueous phase extracted with further ethyl acetate (50mℓ). The combined organic extracts were washed with 8% sodium bicarbonate solution (50mℓ), dried ($Na_2SO_4$) and concentrated to yield the crude product which was purified on a column of 'flash' silica (Merck 9385; 15x3.5cm), elution with toluene/ethanol/0.88$ONH_3$ (39:10:1) affording a colourless viscous oil, which when taken up in dry ether (ca 10mℓ) deposited the title compound as a colourless powder (0.26g) m.p. 104-105°.
T.l.c. $SiO_2$ (Toluene:EtOH:0.88$ONH_4OH$ 39:10:1) Rf 0.24.

Example 5

The following are examples of suitable formulations of compounds of the invention. The term "active ingredient" is used herein to represent a compound of the invention.

Tablets (Direct Compression)

| | mg/tablet |
|---|---|
| Active ingredient | 2.0 |
| Microcrystalline Cellulose USP | 196.5 |
| Magnesium Stearate BP | 1.5 |
| Compression weight | 200.0 |

The active ingredient is sieved through a suitable sieve, blend with the excipients and compressed using 7mm diameter punches.

Tablets of other strengths may be prepared by altering the ratio of active ingredient to microcrystalline cellulose of the compression weight and using punches to suit.

The tablets may be film coated with suitable film forming materials, such as hydroxypropylmethylcellulose, using standard techniques. Alternatively the tablets may be sugar coated.

Metered Dose Pressurized Aerosol (Suspension Aerosol)

| | mg/metered dose | Per can |
|---|---|---|
| Active ingredient micronised | 0.100 | 26.40mg |
| Oleic Acid BP | 0.100 | 2.64mg |
| Trichlorofluoromethane BP | 23.64 | 5.67g |
| Dichlorodifluoromethane BP | 61.25 | 14.70g |

The active ingredient is micronised in a fluid energy mill to a fine particle size range. The oleic acid is mixed with the trichlorofluoromethane at a temperature of 10-15°C and the micronise drug is mixed into the solution with a high shear mixer. The suspension is metered into aluminium aerosol cans and suitable metering valves delivered 85mg of suspension are crimped onto the cans and the dichlorodifluoromethane is pressure filled into the can through the valves.

Inhalation Cartridges

| | | mg/cartridge |
|---|---|---|
| Active ingredient micronised | | 0.200 |
| Lactose BP | to | 25.0 |

The active ingredient is micronised in a fluid energy mill to a fine particle size range prior to blending with normal tabletting grade lactose in a high energy mixer. The powder blend is filled in No. 3 hard gelatin capsules on a suitable encapsulating machine. Th contents of the cartridges are administered using a a powder inhaler such as the Glaxo Rotahaler.

## Claims

1. Compounds of the general formula (I)

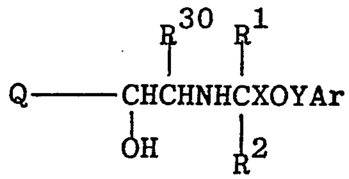

(I)

wherein

Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^4$ (where $R^3$ and $R^4$ each represents a hydrogen atom, or a $C_{1-4}$ alkyl group, or $-NR^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy,$C_{1-4}$alkoxy or $-NR^3R^4$), $-SR^9$ is a hydrogen atom, or a $C_{1-4}$ alkyl or phenyl group), $-SOR^9$, or -CN, and q represents an integer from 0 to 3], or $-O(CH_2)_tR^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;

$R^1$ and $R^2$ each represent a hydrogen atom or a $C_{1-3}$ alkyl group, with the proviso that the sum total of carbon atoms in $R^1$ and $R^2$ is not more than 4;

$R^{30}$ represents a hydrogen atom or a $C_{1-3}$ alkyl group;

X represents a $C_{2-8}$ alkylene chain optionally substituted by one or two $C_{1-3}$ alkyl groups, or, when one carbon atom is substituted by two methyl groups, they may be linked to form an ethylene group, and Y represents a $C_{1-7}$ alkylene chain optionally substituted by one or two $C_{1-3}$ alkyl groups, or, when one carbon atom is substituted by two alkyl groups, they may be linked to form an alkylene group; with the proviso that the sum total of carbon atoms in X and Y is 4 to 12; and with the further proviso that at least one of X and Y is substituted by one or two $C_{1-3}$ alkyl groups or an alkylene group;

Q represents

(a) 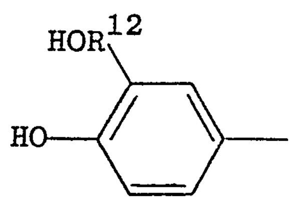

(where $R^{12}$ is a straight or branched $C_{1-3}$ alkylene group),

(b) 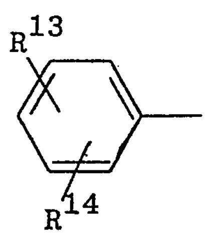

(where one of $R^{13}$ and $R^{14}$ is a hydroxy group and the other is a hydrogen or halogen atom or a hydroxy group),

(c) 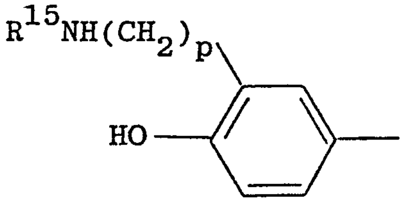

[where $R^{15}$ is a group $R^{16}CO-$, $R^{16}NHCO-$, $R^{16}R^{17}NSO_2-$ or $R^{18}SO_2-$ (where $R^{16}$ and $R^{17}$ is each a

hydrogen atom or a $C_{1-3}$ alkyl group and $R^{18}$ is a $C_{1-3}$ alkyl group) and p is an integer 0 or 1],

(d) 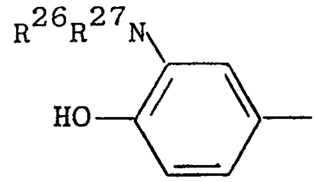

(where $R^{26}$ and $R^{27}$ is each a hydrogen atom or a $C_{1-3}$ alkyl group or, when $R^{26}$ is a hydrogen atom, $R^{27}$ may also be a $C_{1-4}$ alkoxycarbonyl group),

(e) 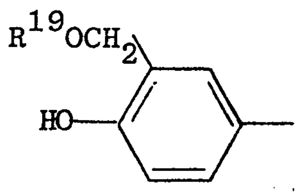

(where $R^{19}$ is a $C_{1-3}$ alkyl group),

(f) 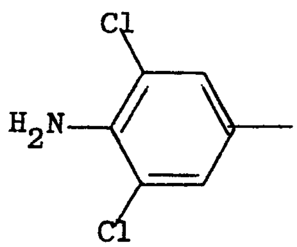

(g) 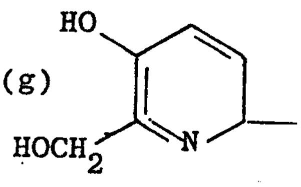

(h) 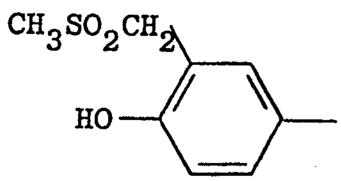

(i) 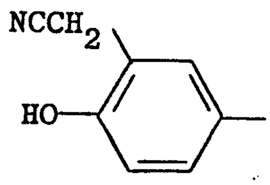

or

(j) 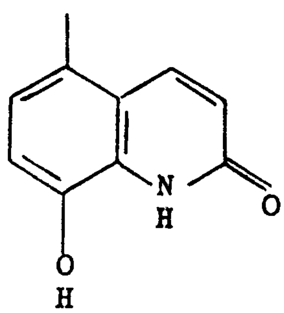

or

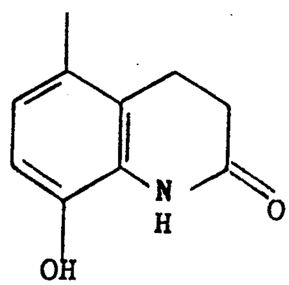

;

and physiologically acceptable slats and solvates thereof.

2. Compounds as claimed in claim 1 in which $R^1$ and $R^2$ each independently represent a hydrogen atom or a methyl group.

3. Compounds as claimed in claim 1 or 2 in which the sum total of carbon atoms in X and Y is 7, 8 or 9.

4. Compounds as claimed in any of claims 1 to 3 in which Y is $-(CH_2)_2-$, $-CH_2\underset{CH_3}{\underset{|}{CH}}-$, $-\underset{CH_3}{\underset{|}{CH}}(CH_2)_2-$ or

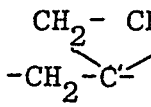 and

and X is $-(CH_2)_5$ or

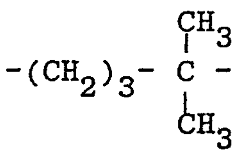 .

5. Compounds as claimed in any of claims 1 to 4 in which Ar is phenyl; or Ar is phenyl substituted by one or more substituents selected from chlorine, bromine, iodine, fluorine, methyl, ethyl, ethoxy, $-(CH_2)_qR$ [where R is hydroxy, methoxy, amino, methylamino, ethylamino, dimethylamino, diethylamino, morpholino, piperidino, piperazino, N-methylpiperazino, $-NHCOR^6$ (where $R^6$ is hydrogen or $C_{1-4}$ alkyl, $C_{1-4}$ alkoxy, phenyl, amino or N,N-dimethylamino), $-N(CH_3)COCH_3$, $-NR^5SO_2R^7$ (where $R^5$ represents a hydrogen atom or a methyl group and $R^7$ represents methyl, butyl, phenyl, amino or dimethylamino), $-COOH$, $COOCH_3$, $-COOCH_2CH_2CH_3$, $-CONH_2$, $-CON(CH_3)_2$, $-CON(CH_2CH_3)_2$, $-CON(CH_2CH_2CH_3)_2$, $-SR^9$ (where $R^9$ represents methyl, ethyl, or phenyl) $-SOCH_3$, $-SO_2CH_3$, or CN, and q is zero, 1, 2 or 3], $-NO_2$, $-O(CH_2)_2OH$, $-O(CH_2)_3OH$, $-O(CH_2)_2OCH_3$, or $-O(CH_2)_2OCH_2CH_3$.

6. Compounds as claimed in any of claims 1 to 5 in which Q represents

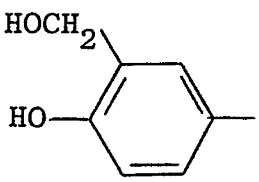 , 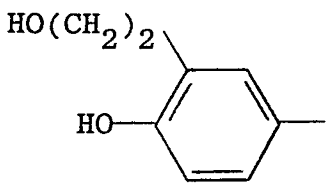 , 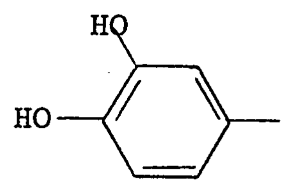 ,

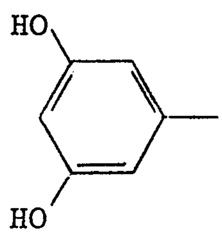 , 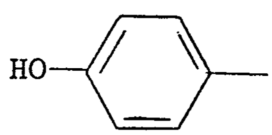 , 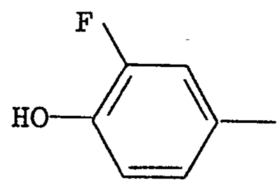 ,

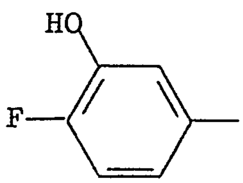 , 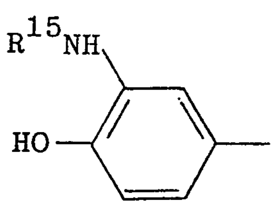

$CH_3CO-$, $NH_2CO-$, $(CH_3)_2NSO_2-$ or $CH_3SO_2-$),

(where $R^{15}$ is $HCO-$,

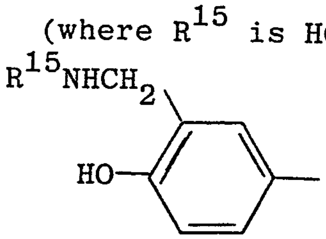

(where $R^{15}$ is as just defined),

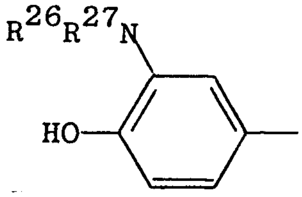

(where $R^{26}$ is hydrogen and $R^{27}$ is methyl),

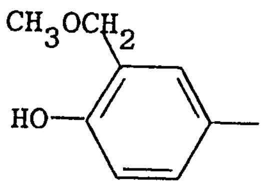 , 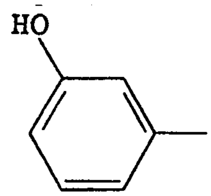

or a group of type (f), (g), (h), (i) or (j).

7. Compounds as claimed in any of claims 1 to 6 in which $R^1$, $R^2$ and $R^{30}$ are each hydrogen atoms, Ar is a phenyl group, Q is the group

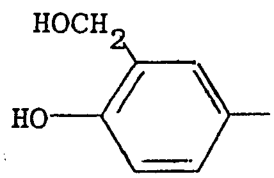 ,

X is $-(CH_2)_5-$ and Y is 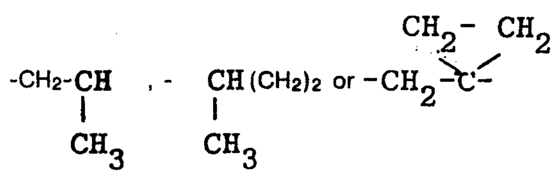 .

8. A process for the preparation of compounds as claimed in any of claims 1 to 7 or a physiologically acceptable salt or solvate thereof which comprises:

(1a) for the preparation of a compound of formula (I) in which $R^1$ is a hydrogen atom, alkylating an amino of general formula (II)

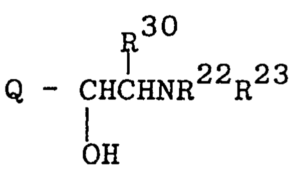 (II)

(wherein $R^{22}$ is a hydrogen atom or a protecting group, $R^{23}$ is a hydrogen atom, and any hydroxyl group(s) or amino group in the group Q may be protected) with an alkylating agent of general formula (III)

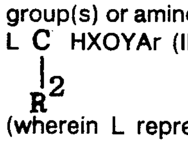 (III)

(wherein L represents a leaving group) followed, if necessary, by removal of any protecting group present; or

(1b) for the preparation of a compound of formula (I) in which $R^1$ is a hydrogen atom, alkylating an amino of general formula (II) as defined above except that $R^{23}$ is a hydrogen atom or a group convertible thereto under the reaction conditions, with a compound of general formula (IV)

$R^2COXOYAr$ (IV)

in the presence of a reducing agent followed, if necessary, by removal of any protecting groups present; or

(2) deprotecting a protected intermediate of general formula (VI)

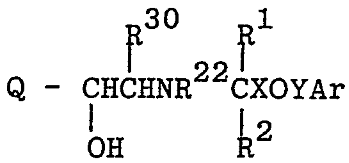

(VI)

(wherein $R^{22}$ and Q are as defined in claim 1 except that either $R^{22}$ is a protecting group and/or at least one of its hydroxyl or amino group(s) in Q is protected); or
(3) reducing an intermediate of general formula (VIII)

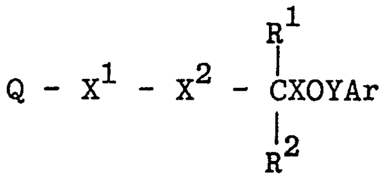

(VIII)

wherein Q is as defined in claim 1; $X^1$ is -CH(OH)- or a group convertible thereto by reduction; $X^2$ is $-CHR^{30}NR^{22}-$ (where $R^{22}$ is hydrogen or a protecting group) or a group convertible thereto by reduction; at least one of $X^1$ and $X^2$ representing a reducible group and/or Q containing a reducible group, followed, if necessary, by removal of any protecting group present; and if desired, converting the resulting compound of general formula (I) or a salt thereof into a physiologically acceptable salt or solvate thereof.

9. A pharmaceutical composition comprising at least one compound of general formula (I) as defined in any of claims 1 to 7 or a physiologically acceptable salt of solvate thereof, together with a physiologically acceptable carrier or excipient.

CLAIMS FOR: AUSTRIA, GREECE, SPAIN

1. A process for the preparation of compounds of the general formula (I)

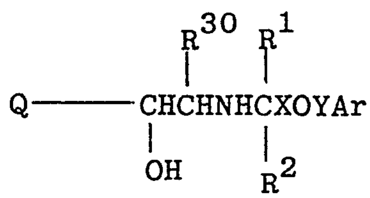

(I)

wherein
Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^1$ (where $R^3$ and $R^4$ each represents a hydrogen atom, or a $C_{1-4}$ alkyl group, or $-NR^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$alkoxy or $-NR^3R^4$), $-SR^9$ is a hydrogen atom, or a $C_{1-4}$ alkyl or phenyl group), $-SOR^9$, $SO_2R^9$ or -CN, and q represents an integer from 0 to 3], or $-O(CH_2)_tR^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;
$R^1$ and $R^2$ each represent a hydrogen atom or a $C_{1-3}$ alkyl group, with the proviso that the sum total of carbon atoms in $R^1$ and $R^2$ is not more than 4;
$R^{30}$ represents a hydrogen atom or a $C_{1-3}$ alkyl group;
X represents a $C_{2-8}$ alkylene chain optionally substituted by one or two $C_{1-3}$ alkyl groups, or, when one carbon atom is substituted by two methyl groups, they may be linked to form an ethylene group, and Y represents a $C_{1-7}$ alkylene chain optionally substituted by one or two $C_{1-3}$ alkyl groups, or, when one carbon atom is substituted by two alkyl groups, they may be linked to form an alkylene group; with the proviso that the sum total of carbon atoms in X and Y is 4 to 12; and with the further proviso that at least one of X and Y is substituted by one or two $C_{1-3}$ alkyl groups or an alkylene group;
Q represents

(a) 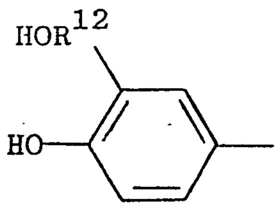

(where $R^{12}$ is a straight or branched $C_{1-3}$ alkylene group),

(b) 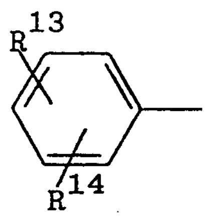

(where one of $R^{13}$ and $R^{14}$ is a hydroxy group and the other is a hydrogen or halogen atom or a hydroxy group),

(c) 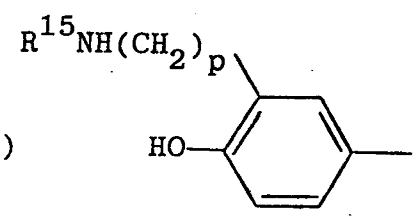

[where $R^{15}$ is a group $R^{16}CO$-, $R^{16}NHCO$-, $R^{16}R^{17}NSO_2$- or $R^{18}SO_2$- (where $R^{16}$ and $R^{17}$ is each a hydrogen atom or a $C_{1-3}$ alkyl group and $R^{18}$ is a $C_{1-3}$ alkyl group) and p is an integer 0 or 1],

(d) 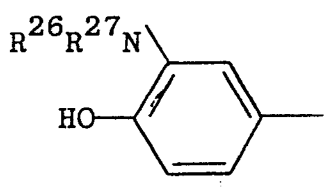

(where $R^{26}$ and $R^{27}$ is each a hydrogen atom or a $C_{1-3}$ alkyl group or, when $R^{26}$ is a hydrogen atom, $R^{27}$ may also be a $C_{1-4}$ alkoxycarbonyl group),

(e) 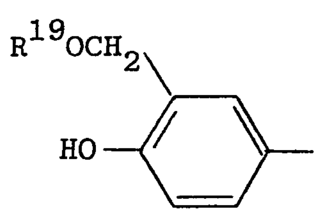

(where $R^{19}$ is a $C_{1-3}$ alkyl group),

(f) 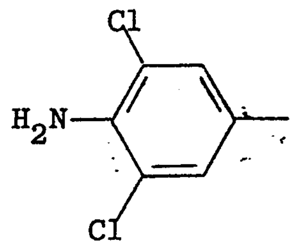 (g) 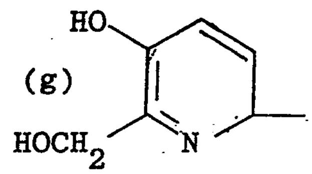

(h) 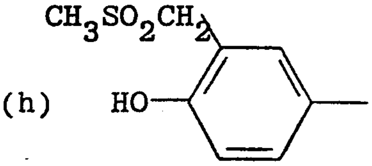 (i) 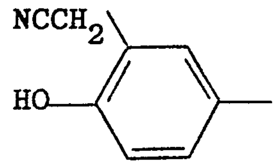

or

(j) 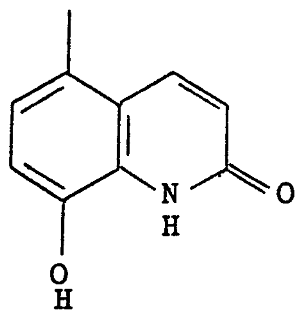 or 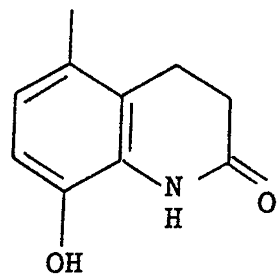 ;

and physiologically acceptable salts and solvates thereof which comprises:

(1a) for the preparation of a compound of formula (I) in which $R^1$ is a hydrogen atom, alkylating an amino of general formula (II)

$$\begin{array}{l} \quad\ \ R^{30} \\ \quad\ \ | \\ Q - CHCHNR^{22}R^{23} \\ \quad\ | \\ \quad\ OH \end{array} \qquad (II)$$

(wherein $R^{22}$ is a hydrogen atom or a protecting group, $R^{23}$ is a hydrogen atom, and any hydroxyl group(s) or amino group in the group Q may be protected) with an alkylating agent of general formula (III)

$$\begin{array}{l} LC\ \ HXOYAr \\ | \\ R^2 \end{array} \quad (III)$$

(wherein L represents a leaving group) followed, if necessary, by removal of any protecting group present; or

(1b) for the preparation of a compound of formula (I) in which $R^1$ is a hydrogen atom, alkylating an amino of general formula (II) as defined above except that $R^{23}$ is a hydrogen atom or a group convertible thereto under the reaction conditions, with a compound of general formula (IV)

$R^2COXOYAr$ (IV)

in the presence of a reducing agent followed, if necessary, by removal of any protecting groups present; or

(2) deprotecting a protected intermediate of general formula (VI)

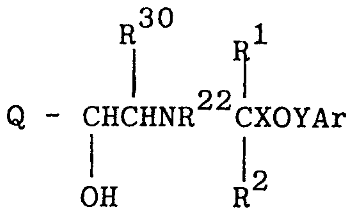

(VI)

(wherein $R^{22}$ and Q are as defined above except that either $R^{22}$ is a protecting group and/or at least one of its hydroxyl or amino group(s) in Q is protected); or

(3) reducing an intermediate of general formula (VIII

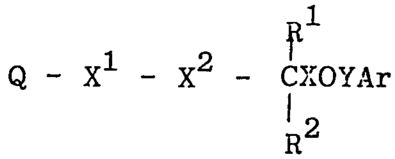

(VIII)

wherein Q is as defined in claim 1; $X^1$ is -CH(OH)- or a group convertible thereto by reduction; $X^2$ is $-CHR^{30}NR^{22}-$ (where $R^{22}$ is hydrogen or a protecting group) or a group convertible thereto by reduction; at least one of $X^1$ and $X^2$ representing a reducible group and/or Q containing a reducible group, followed, if necessary, by removal of any protecting group present; and if desired, converting the resulting compound of general formula (I) or a salt thereof into a physiologically acceptable salt or solvate thereof.

2. A process as claimed in claim 1 for the production of compounds in which $R^1$ and $R^2$ each independently represent a hydrogen atom or a methyl group.

3. A process as claimed in claim 1 or 2 for the production of compounds in which the sum total of carbon atoms in X and Y is 7, 8 or 9.

4. A process as claimed in any of claims 1 to 3 for the production of compounds in which Y is $-(CH_2)_2-$, $-CH_2CH-$, $-CH(CH_2)_2-$ or

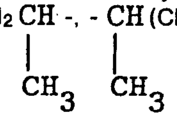

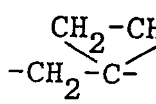
and

and X is $-(CH_2)_5$ or

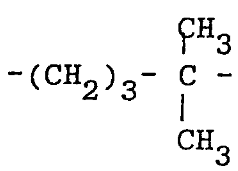

5. A process as claimed in any of claims 1 to 4 for the production of compounds in which Ar is phenyl; or Ar is phenyl substituted by one or more substituents selected from chlorine, bromine, iodine, fluorine, methyl, ethyl, ethoxy, $-(CH_2)_qR$ [where R is hydroxy, methoxy, amino, methylamino, ethylamino, dimethylamino, diethylamino, morpholino, piperidino, piperazino, N-methylpiperazino, $-NHCOR^6$ (where $R^6$ is hydrogen or $C_{1-4}$ alkyl, $C_{1-4}$ alkoxy, phenyl, amino or N,N-dimethylamino), $-N(CH_3)COCH_3$, $-NR^5SO_2R^7$ (where $R^5$ represents a hydrogen atom or a methyl group and $R^7$ represents methyl, butyl, phenyl, amino or dimethylamino), -COOH, $COOCH_3$ - $COOCH_2CH_2CH_3$, $-CONH_2$, $-CON(CH_3)_2$, $-CON(CH_2CH_3)_2$, $-CON(CH_2CH_2CH_3)_2$, $-SR^9$ (where $R^9$ represents methyl, ethyl, or phenyl) $-SOCH_3$, $-SO_2CH_3$, or CN, and q is zero, 1, 2 or 3], $-NO_2$, $-O(CH_2)_2OH$, $-O(CH_2)_3$, $-O(CH_2)_2OCH_3$, or $-O(CH_2)_2OCH_2CH_3$.

6. A process as claimed in any of claims 1 to 5 for the production of compounds in which Q represents

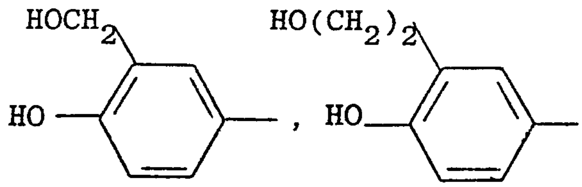 , , 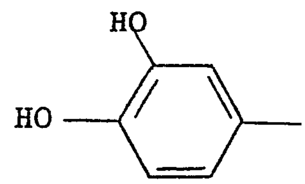 ,

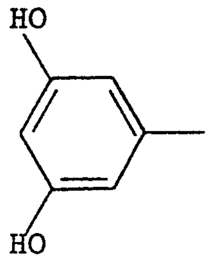 , 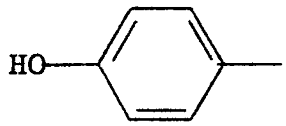 , 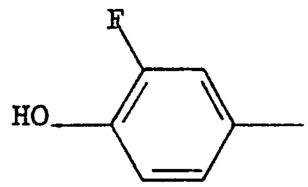 ,

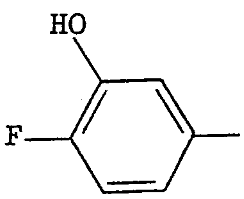 , 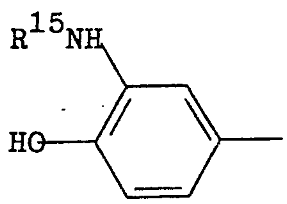

$CH_3CO$-, $NH_2CO$-, $(CH_3)_2NSO_2$- or $CH_3SO_2$-), (where $R^{15}$ is HCO-,

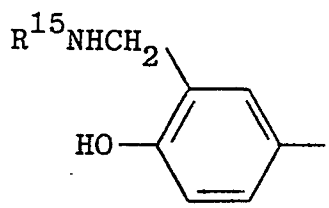

(where $R^{15}$ is as just defined),

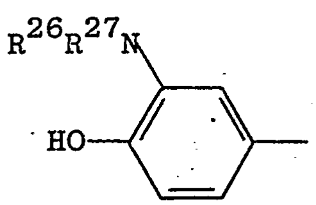

(where $R^{26}$ is hydrogen and $R^{27}$ is methyl),

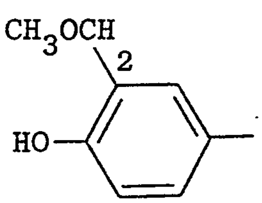 , 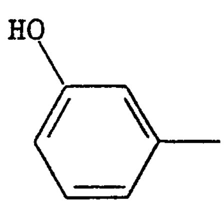 or a group of type (f),

or a group of type (f), (g), (h), (i) or (j).

7. A process as claimed in any of claims 1 to 6 for the production of compounds in which $R^1$, $R^2$ and $R^{30}$ are each hydrogen atoms, Ar is a phenyl group, Q is the group

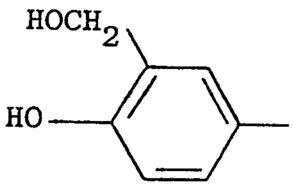

X is $-(CH_2)_5-$ and Y is $-CH_2-$ 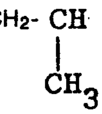 -, - 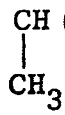 $(CH_2)_2$ or